# EUROPEAN PATENT APPLICATION

(11) **EP 4 356 970 A1**
(43) Date of publication of application: **24.04.2024**
(21) Application number: 22824240.0
(22) Date of filing: 15.06.2022
(51) Int. Cl.: A61P 35/00, A61K 35/17, C12N 5/10, C12N 15/62, C07K 19/00, C12N 15/86

(54) **PREPARATION AND APPLICATION OF LOX1-BASED CHIMERIC ANTIGEN RECEPTOR IMMUNE CELL**

(30) Priority: 16.06.2021 CN 202110665857
(71) Applicant: West China Hospital of Sichuan University, Chengdu, Sichuan 610041 (CN)
(72) Inventor: ZHAO, Xudong, Chengdu, Sichuan 610041 (CN); SUN, Bin, Chengdu, Sichuan 610041 (CN)
(74) Representative: Santoro, Sofia
(86) International application number: PCT/CN2022/098908
(87) International publication number: WO 2022/262764

(57) **Abstract**

A preparation and an application of a chimeric antigen receptor immune cell constructed on the basis of a C-type lectin superfamily low-density lipoprotein receptor (LOX1). Specifically provided is a chimeric antigen receptor (CAR) modified on the basis of LOX1. The CAR contains an extracellular binding domain capable of specifically targeting LOX1 receptors such as heat shock protein, oxidized low-density lipoprotein (oxLDL) and phosphatidylserine. The CAR immune cell has strong specificity and target affinity, and therefore has strong target cell killing capability and high safety.

## Description

### Technical field

The present invention belongs to the field of immune cell treatment, and specifically relates to preparation and application of a chimeric antigen receptor immune cell constructed on basis of LOX1.

### Background

Tumor is the second major disease that threatens human health. In 2018, there were 18,100,000 new cancer patients and 9,500,000 tumor deaths worldwide. It is estimated that by 2040, there will be 29,500,000 new cases and 16,400,000 deaths of cancer per year. Although conventional tumor treatment methods such as radiotherapy, chemotherapy, and surgical resection are able to prolong the survival of cancer patients, they are still constrained by the decline in life quality of patients and high risk of recurrence.

At the 2012 Annual Conference of the International Society for Cellular Therapy, it was pointed out that bioimmunotherapy has become the fourth cancer treatment method after surgery, radiotherapy and chemotherapy, and will become a must-have method for future cancer treatment. Chimeric Antigen Receptor-T (CAR-T) cells refer to T cells that are genetically modified to be able to recognize specific target antigens in a non MHC-restricted manner, with continuous activation and amplification. The structure of CAR comprises a tumor associated antigen binding region, an extracellular hinge region, a transmembrane region and an intracellular signaling region. At present, CART therapy has shown strong killing ability in hematological malignancies, but the application of CART therapy in solid tumors is limited due to tumor heterogeneity, lack of tumor specific antigens, and tumor immunosuppressive micro environment.

Heat shock response is a self-protection mechanism produced by cells or tissues in the face of various stress stimuli, often accompanied by upregulation of heat shock proteins (HSPs) expression. HSP70, as the most important heat shock protein in humans, is low expressed and localized within cells under normal physiological conditions, but overexpressed in tumor cells to promote cell proliferation, inhibit cell aging, and resist stress-induced cell apoptosis. Inhibiting HSP70 in tumor cells can lead to cell death and the formation of transplanted tumors in nude mouse. HSP70 is highly expressed in most tumor samples, which can be used as a molecular marker of early prostate cancer and liver cancer, and is related to the prognosis of various tumors such as acute myeloid leukemia, breast cancer, endometrial cancer and rectal cancer. Methods targeting HSP70 expression, such as shRNA, CRISPR/Cas9 knockout, aptamers, inhibitors and the like are expected to be new approaches for cancer treatment. However, due to the complex heat shock protein family in eukaryotes, and the certain functional compensation among members of the heat shock protein family, other members of the HSPs family may compensate for the effect of HSP70 while the above methods reduce the expression of HSP70.

Therefore, there is an urgent need to develop a chimeric antigen receptor immune cell targeting heat shock protein and a therapeutic method using the same in this field.

### Summary of the invention

The purpose of the invention is to provide a chimeric antigen receptor immune cell targeting LOX1 receptor and the preparation and application method thereof.

In the first aspect of the present invention, it provides a chimeric antigen receptor (CAR) modified based on C-type lectin superfamily low-density lipoprotein receptor (LOX1), wherein the CAR comprises an extracellular binding domain that specifically binds to a LOX1 receptor.

In another preferred embodiment, the LOX1 receptor is selected from a group consisting of: a heat shock protein, oxidized low-density lipoprotein (oxLDL) and phosphatidylserine.

In another preferred embodiment, the extracellular binding domain comprises an amino acid sequence derived from LOX1.

In another preferred embodiment, the extracellular binding domain comprises LOX1 protein or a fragment thereof.

In another preferred embodiment, the fragment of LOX1 protein comprises an extracellular domain of the LOX1 protein.

In another preferred embodiment, the fragment of LOX1 protein comprises an CTLD domain of the LOX1 protein.

In another preferred embodiment, the LOX1 protein or the fragment thereof specifically binds to a LOX1 receptor including heat shock protein.

In another preferred embodiment, the heat shock protein is selected from a group consisting of: HSP70, HSP60, HSP90, and a combination thereof.

In another preferred embodiment, the heat shock protein is a heat shock protein located on the cell membrane (or membrane-bound) (mHSP).

In another preferred embodiment, the heat shock protein is selected from a group consisting of: mHSP70, mHSP60, mHSP90, and a combination thereof.

In another preferred embodiment, the heat shock protein is HSP70, preferably mHSP70.

In another preferred embodiment, the heat shock protein is derived from human or a non-human mammal.

In another preferred embodiment, the non-human mammal includes: a rodent (such as a rat, a mouse), a primate (such as a monkey); and preferably is a primate.

In another preferred embodiment, the heat shock protein is of human or monkey origin.

In another preferred embodiment, the heat shock protein is of human origin.

In another preferred embodiment, the LOX1 protein or the fragment thereof has an amino acid sequence as shown in SEQ ID NO: 1, or has an amino acid sequence as shown in positions 1-273 (preferably positions 38-273, more preferably positions 58-273, and more preferably positions 151-256) of SEQ ID NO: 1.

In another preferred embodiment, the LOX1 protein or the fragment thereof comprises a c-type lectin domain (CTLD), the amino acid sequence of which corresponds to positions 151-265 of the sequence shown in SEQ ID NO: 1.

In another preferred embodiment, the amino acid sequence of the LOX1 protein or the fragment thereof is selected from a group consisting of:
(i) a sequence as shown in positions 58-273 of the sequence of SEQ ID NO: 1; and
(ii) an amino acid sequence obtained by replacing, deleting, altering or inserting one or more amino acid residues, or adding 1-30 amino acid residues, preferably 1-10 amino acid residues, more preferably 1-5 amino acid residues on the basis of the sequence shown in positions 58-273 of the sequence of SEQ ID NO: 1; wherein the obtained amino acid sequence has a sequence identity of ≥ 85% (preferably ≥ 90%, more preferably ≥ 95%, such as ≥ 96%, ≥ 97%, ≥ 98%, or ≥ 99%) with the sequence as shown in positions 58-273 of SEQ ID NO: 1; and the obtained amino acid sequence has the same or similar function as the sequence in (i).

In another preferred embodiment, the structure of the CAR is shown in the following Formula I:

L-EB-H-TM-C-CD3ζ-RP (I)

wherein,
each "-" is independently a linking peptide or peptide bond;
L is absent or a signal peptide sequence;
EB is an extracellular binding domain;
H is absent or a hinge region;
TM is a transmembrane domain;
C is absent or a co-stimulatory signaling molecule;
CD3ζ is a cytoplasmic signal transduction sequence derived from CD3ζ;
RP is absent or a reporter protein.

In another preferred embodiment, the reporter protein RP further comprises a self splicing recognition site located at the N-terminus, preferably a T2A sequence.

In another preferred embodiment, the reporter protein RP is a fluorescent protein.

In another preferred embodiment, the reporter protein RP is a mKate2 red fluorescent protein.

In another preferred embodiment, the amino acid sequence of the mKate2 red fluorescent protein is shown in SEQ ID NO: 2.

In another preferred embodiment, L is a signal peptide of a protein selected from a group consisting of CD8, CD28, GM-CSF, CD4, CD137 and a combination thereof.

In another preferred embodiment, L is a signal peptide derived from CD8.

In another preferred embodiment, the amino acid sequence of L is shown in SEQ ID NO: 3.

In another preferred embodiment, H is a hinge region of a protein selected from a group consisting of CD8, CD28, CD137 and a combination thereof.

In another preferred embodiment, H is a hinge region derived from CD8.

In another preferred embodiment, the amino acid sequence of H is shown in SEQ ID NO: 4.

In another preferred embodiment, TM is a transmembrane region of a protein selected from a group consisting of CD28, CD3 epsilon, CD45, CD4, CD5, CD8, CD9, CD16, CD22, CD33, CD37, CD64, CD80, CD86, CD134, CD137, CD154 and a combination thereof.

In another preferred embodiment, TM is a transmembrane region derived from CD28.

In another preferred embodiment, the amino acid sequence of TM is shown in SEQ ID NO: 5.

In another preferred embodiment, C is a co-stimulatory signaling molecule of a protein selected from a group consisting of OX40, CD2, CD7, CD27, CD28, CD30, CD40, CD70, CD134, 4-1BB (CD137), PD1, Dap10, CDS, ICAM-1, LFA-1 (CD11a/CD18), ICOS (CD278), NKG2D, GITR, TLR2 and a combination thereof.

In another preferred embodiment, C is a co-stimulatory signaling molecule derived from 4-1BB.

In another preferred embodiment, the amino acid sequence of C is shown in SEQ ID NO: 6.

In another preferred embodiment, the amino acid sequence of the cytoplasmic signal transduction sequence derived from CD3ζ is shown in SEQ ID NO: 7.

In another preferred embodiment, the amino acid sequence of the chimeric antigen receptor (CAR) is shown in SEQ ID NO: 8.

In the second aspect of the present invention, it provides a nucleic acid molecule encoding the chimeric antigen receptor according to the first aspect of the present invention.

In the third aspect of the present invention, it provides a vector which comprises the nucleic acid molecule according to the second aspect of the present invention.

In another preferred embodiment, the vector is selected from a group consisting of DNA, RNA, plasmid, lentiviral vector, adenoviral vector, retroviral vector, transposon and a combination thereof.

In another preferred embodiment, the vector is a lentiviral vector.

In another preferred embodiment, the vector is selected from a group consisting of pTomo lentiviral vector, plenti, pLVTH, pLJM1, pHCMV, pLBS.CAG, pHR, pLV, etc..

In another preferred embodiment, the vector includes a pTomo lentiviral vector.

In another preferred embodiment, the vector further comprises an element selected from a group consisting of: a promoter, a transcription enhancing element WPRE, a long terminal repeat sequence LTR, etc.

In another preferred embodiment, the vector comprises an nucleotide sequence shown in SEQ ID NO: 9.

In the fourth aspect of the present invention, it provides a host cell comprising the vector according to the third aspect of the present invention, or having the exogenous nucleic acid molecule according to the second aspect of the present invention integrated into the chromosome, or expressing the CAR according to the first aspect of the present invention.

In the fifth aspect of the present invention, it provides an engineered immune cell comprising the vector according to the third aspect of the present invention, or having the exogenous nucleic acid molecule according to the second aspect of the present invention integrated into the chromosome, or expressing the CAR according to the first aspect of the present invention.

In another preferred embodiment, the immune cell is selected from a group consisting of: T cell, NK cell, NKT cell, and macrophage.

In another preferred embodiment, the engineered immune cell is a chimeric antigen receptor T cell (CAR-T cell) or a chimeric antigen receptor NK cell (CAR-NK cell).

In another preferred embodiment, the engineered immune cell is a CAR-T cell.

In the sixth aspect of the present invention, it provides a method for the preparation of the engineered immune cell according to the fifth aspect of the present invention, which comprises a step of: transducing the nucleic acid molecule according to the second aspect of the present invention or the vector according to the third aspect of the present invention into an immune cell, thereby obtaining the engineered immune cell.

In another preferred embodiment, the method further comprises a step of detecting the function and effectiveness of the obtained engineered immune cell.

In the seventh aspect of the present invention, it provides a pharmaceutical composition comprising the CAR according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, and a pharmaceutically acceptable carrier, diluent or excipient.

In another preferred embodiment, the formulation is a liquid preparation.

In another preferred embodiment, the dosage form of the formulation is injection.

In another preferred embodiment, the concentration of the engineered immune cell in the formulation is 1×10³-1×10⁸ cells/ml, and preferably 1×10⁴-1×10⁷ cells/ml..

In the eighth aspect of the present invention, it provides a use of the CAR according to the first aspect of the present invention, the nucleic acid molecule according to the second aspect of the present invention, the vector according to the third aspect of the present invention, the host cell according to the fourth aspect of the present invention, and/or the engineered immune cell according to the fifth aspect of the present invention, in the preparation of a medicament or a formulation for preventing and/or treating a disease related to abnormal expression of LOX1 receptor.

In another preferred embodiment, the LOX1 receptor includes but is not limited to mHSP70, mHSP60, mHSP90, oxLDL and the like.

In another preferred embodiment, the disease related to abnormal expression of LOX1 receptor includes but is not limited to a tumor, aging, obesity, cardiovascular disease, diabetes, neurodegenerative disease, infectious disease and the like.

In another preferred embodiment, the disease related to abnormal expression of LOX1 receptor includes a disease related to abnormal expression of membrane-bound HSP70 (mHSP70).

In another preferred embodiment, the disease related to abnormal expression of mHSP70 includes tumor, aging, cardiovascular disease, obesity and the like.

In another preferred embodiment, the disease is a malignant tumor with high expression of mHSP70.

In another preferred embodiment, the tumor includes a hematological tumor and solid tumor.

In another preferred embodiment, the hematological tumor is selected from a group consisting of: acute myeloid leukemia (AML), multiple myeloma (MM), chronic lymphocytic leukemia (CLL), acute lymphocytic leukemia (ALL), diffuse large B cell lymphoma (DLBCL) and a combination thereof.

In another preferred embodiment, the solid tumor is selected from a group consisting of: breast cancer, gastric cancer, hepatobiliary cancer, colorectal cancer, bladder cancer, non-small cell lung cancer, ovarian cancer and esophageal cancer, glioma, lung cancer, pancreatic cancer, prostate cancer and a combination thereof.

In another preferred embodiment, the tumor is selected from a group consisting of: acute myeloid leukemia, breast cancer, endometrial cancer, and rectal cancer.

In the ninth aspect of the present invention, it provides a use of the engineered immune cell according to the fifth aspect of the present invention or the pharmaceutical composition according to the seventh aspect of the present invention, in preventing and/or treating a tumor or cancer.

In the tenth aspect of the present invention, it provides a method of treating a disease which comprises: administering an effective amount of the engineered immune cell according to the fifth aspect of the present invention, or the pharmaceutical composition of the seventh aspect of the present invention, to a subject in need thereof.

In another preferred embodiment, the disease is related to abnormal expression of a LOX1 receptor.

In another preferred embodiment, the disease is a cancer or tumor.

In another preferred embodiment, the engineered immune cell or the CAR immune cell comprised in the pharmaceutical composition is a cell derived from the subject (an autologous cell).

In another preferred embodiment, the engineered immune cell or the CAR immune cell comprised in the pharmaceutical composition is a cell derived from a healthy individual (an allogeneic cell).

In another preferred embodiment, the method may be utilized in combination with other treatment method.

In another preferred embodiment, said other treatment method includes chemotherapy, radiotherapy, targeted therapy and other methods.

It should be understood that, within the scope of the present invention, the technical features specifically described above and below (such as the Examples) can be combined with each other, thereby constituting a new or preferred technical solution which needs not be described one by one.

### Description of Figure

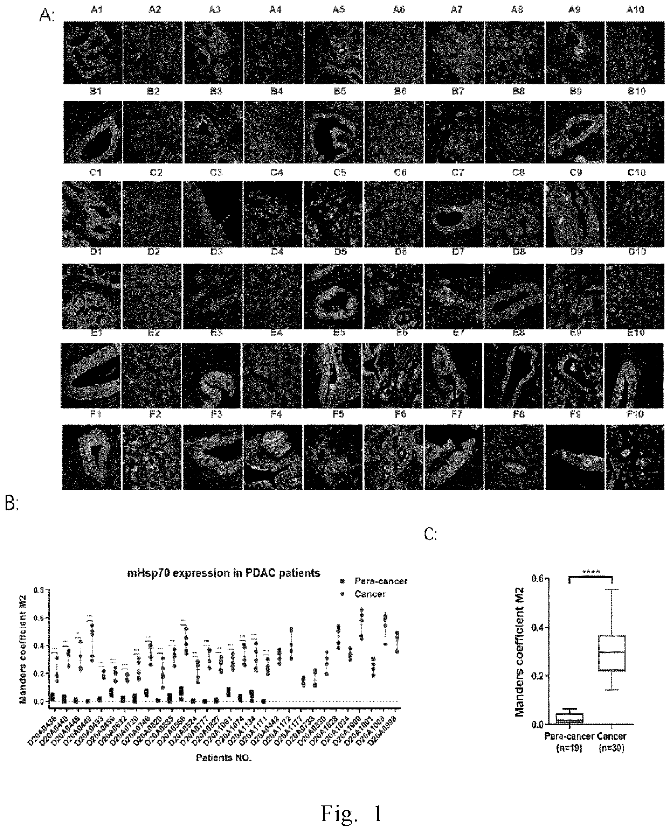

Figure 1 shows the co-localization analysis of HSP70 and β-catenin in pancreatic cancer tissue. A shows the immunohistochemical staining of HSP70 (red) and β-Catenin (green) in a pancreatic cancer tissue chip. A1-E6 represent the corresponding cancer tissue and paracancerous tissue, with odd numbers indicating paracancerous tissues and even numbers indicating cancer tissues. E7-F10 represent pancreatic cancer tissues. B shows the co-localization analysis of HSP70 and β-catenin in each cancer tissue and paracancerous tissue in the pancreatic cancer tissue chips. C shows the co-localization analysis of HSP70 and β-catenin in cancer tissues and paracancerous tissues.

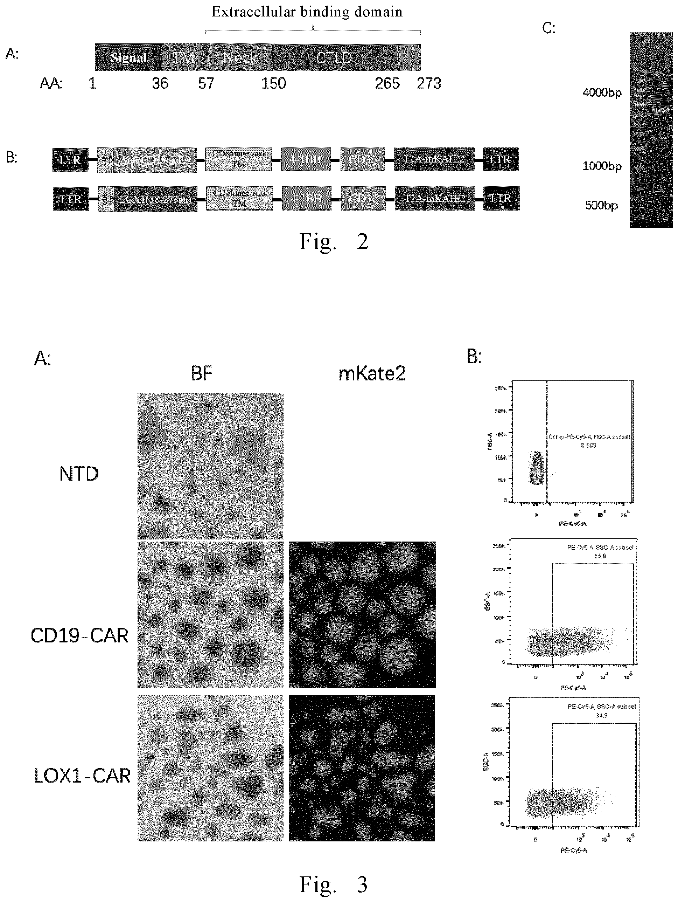

Figure 2 shows a schematic diagram of the construction of LOX1-CAR vector,
   wherein A shows the schematic diagram of LOX1 sequence, wherein 1-36 AA is the signal peptide, 37-57 AA is the transmembrane domain, 58-273 AA is the extracellular domain, and 151-265 AA is the CTLD domain; B shows a schematic diagram of the control plasmid CD19-CAR and LOX1-CAR, wherein the signal peptide, hinge region, and transmembrane region are derived from human CD8 molecules, 4-1BB is derived from human CD137, CD3ζ is derived from human CD3 and mKate2 is a fluorescent marker used for detecting CAR expression; and C shows the identification of pTomo-LOX1-CAR vector by HindIII enzyme digestion.
Figure 3 shows the detection result of CAR transfection efficiency,
   wherein A shows the cell fluorescence expression results of T cells infected with CD19-CAR and LOX1-CAR after 72 hours, where BF represents the bright field and mKate2 is fluorescence expression of CAR; B shows the fluorescence expression result by flow cytometry.
Figure 4 shows the detection result of mHSP70 expression in different tumor cell lines.
Figure 5 shows the *in vitro* killing test results of LOX1-CAR on different tumor cell lines,
   wherein, PANC1, BxPC3, AsPC1 are pancreatic cancer cell lines; PC3 is a prostate cancer cell line; SMMC7721 is a liver cancer cell line; HCT116 is a colorectal cancer cell line, and MCF7 is a breast cancer cell line.
Figure 6 shows the detection result of IFNγ release.
Figure 7 shows LOX1-CART killing pancreatic cancer stem cells. A shows the expression detection of stem cell markers CD133, OCT4 and CXCR4 in AsPC1-CSC and PANC1-CSC. B shows the HSP70 expression in AsPC1-CSC and PANC1-CSC detected by flow cytometry. C and D show the killing of LOX1-CART on AsPC1-CSC and PANC1-CSC (C) and the IFNγ release (D).
Figure 8 shows the effect of HSP70 overexpression on cell killing. MDA-MB231 cells are breast cancer cell lines.
Figure 9 shows the killing effect of LOX1-CAR on normal cells.
Figure 10 shows the inhibition by LOX1-CART on AsPC1-luc xenograft tumors in nude mice. A and B show the growth of subcutaneous xenograft tumors at different time periods after LOX1-CART reinfusion. C shows photos of subcutaneous xenograft tumors after the experiment.
Figure 11 shows that LOX1-CARTs enrich in subcutaneous xenograft tumors and do not cause damage to major organs. A shows the presence of T cells in subcutaneous xenograft tumors, liver, lung, pancreas, spleen, brain, etc. detected by immunohistochemistry. B shows HE staining of subcutaneous xenograft tumors, heart, liver, lung, spleen, kidney, pancreas, brain, etc. after CART reinfusion.
Figure 12 shows the inhibition of AsPC1 metastasis by LOX1-CART. A is the experimental process. B and C show the growth (B) and quantification (C) of subcutaneous xenograft tumors at different time periods after LOX1-CART reinfusion. D shows the statistics of metastatic nodules in lung of the CD19-CART and LOX1-CART groups after the experiment. Growth of subcutaneous xenograft tumors at different time periods after reinfusion.
Figure 13 shows the inhibition of tumor cells in peripheral blood by LOX1-CART. A shows a schematic diagram of the experimental blood collection process. B shows the detection of tumor cells in peripheral blood at different times after CD19-CART and LOX1-CART reinfusion.

### EMBODIMENTS

After extensive and intensive research and widely screening, the inventor has developed the preparation and application of a chimeric antigen receptor immune cell based on LOX1 for the first time. The experimental results show that the CAR-T targeting LOX1 receptor of the present invention has a significant killing effect on target cells and a broad-spectrum anti-tumor cell effect. On this basis, the present invention has been completed.

### Terms

To make it easier to understand the present invention, certain technical and scientific terms are specifically defined below. Unless otherwise clearly defined herein, all other technical and scientific terms used herein have meanings commonly understood by those skilled in the art in the field to which the present invention belongs. Before describing the present invention, it should be understood that the present invention is not limited to the specific methods and experimental conditions described, as such methods and conditions can be changed. It should also be understood that the terms used herein are only intended to describe the specific embodiments and are not intended to be restrictive. The scope of the present invention will be limited only by the accompanying claims.

Unless otherwise defined, all technical and scientific terms used herein have the same meaning as commonly understood by one of ordinary skill in the art to which this invention belongs. As used herein, when used in reference to a specifically recited value, the term "about" means that the value may vary by no more than 1% from the recited value. For example, as used herein, the expression "about 100" includes all values between 99 and 101 (e.g., 99.1, 99.2, 99.3, 99.4, etc.).

As used herein, the term "optional" or "optionally" means that the event or situation described thereafter may occur, but not necessarily.

As used herein, the terms "containing" or "comprising (including)" can be open, semi-closed, and closed. In other words, the term also includes "consisting essentially of," or "consisting of."

"Transduction", "transfection", "transformation" or the terms used herein refer to the process of delivering exogenous polynucleotides to host cells, and transcribing and translating to produce peptide products, including the use of plasmid molecules to introduce exogenous polynucleotides into host cells (such as E. coli).

"Gene expression" or "expression" refers to the process of gene transcription, translation, and post-translational modification to produce RNA or protein products of genes.

"Polynucleotide" refers to the polymerized forms of nucleotides of any length, including deoxyribonucleotides (DNA), ribonucleotides (RNA), and heterozygous sequences and analogues thereof. Polynucleotides can include modified nucleotides, such as methylated or capped nucleotides or nucleotide analogues. The term polynucleotides used herein refers to interchangeable single and double stranded molecules. Unless otherwise specified, polynucleotides in any embodiment described herein include double stranded form and two complementary single strands known or predictable to form a double stranded form.

The substitution of conservative amino acids is known in this field. In some embodiments, potential substituted amino acids are within one or more of the following groups: glycine, alanine; and valine, isoleucine, leucine, and proline; aspartate, glutamic acid; asparagine, glutamine; serine, threonine, lysine, arginine, and histidine; and/or phenylalanine, tryptophan, and tyrosine; methionine and cysteine. In addition, the present invention also provides non-conservative amino acid substitutions that allow amino acids from different functional groups to be substituted.

Those skilled in the art will easily understand the meaning of all parameters, sizes, materials, and configurations described herein. The actual parameters, sizes, materials, and configurations depend on the specific application described herein. Those skilled in the art can understand that embodiments or claims are only provided by example, and within the scope of equivalents or claims, the scope that embodiments of the present invention can cover is not limited to the specific description and claims.

The definitions and all definitions used herein should be understood as exceeding those defined in the dictionary or the documents incorporated by reference.

All references, patents, and patent applications herein of the present invention are incorporated by reference to the subject matter cited, and in some cases may include the entire document.

It should be understood that for any method described in this article that includes more than one step, the order of steps is not necessarily limited to the order described in these embodiments.

To make the disclosure easier to understand, some terms are firstly defined. As used in this application, unless expressly stated otherwise herein, each of the following terms shall have the meanings given below. Other definitions are set forth throughout the application.

The term "about" may refer to a value or composition within an acceptable error range for a particular value or composition as determined by those skilled in the art, which will depend in part on how the value or composition is measured or determined.

The term "administering" refers to the physical introduction of a product of the present invention into a subject using any one of various methods and delivery systems known to those skilled in the art, including intravenous, intramuscular, subcutaneous, intraperitoneal, spinal or other parenteral administration, such as by injection or infusion.

### Heat shock protein family

Heat shock response is a self-protection mechanism produced by cells or tissues in the face of various stress stimuli, often accompanied by upregulation of heat shock proteins (HSPs) expression. According to molecular weight, heat shock proteins mainly include HSP100, HSP90, HSP70, HSP60, HSP40, HSP27, HSP26, etc. HSP70, as the most important heat shock protein in humans, is low expressed and localized within cells under normal physiological conditions, but overexpressed in tumor cells to promote cell proliferation, inhibit cell aging, and resist stress-induced cell apoptosis. Inhibiting HSP70 in tumor cells can lead to cell death and inhibiting the formation of transplanted tumors in nude mouse. HSP70 is highly expressed in most tumor samples, which can be used as a molecular marker of early prostate cancer and liver cancer, and is related to the prognosis of various tumors such as acute myeloid leukemia, breast cancer, endometrial cancer and rectal cancer. Methods targeting HSP70 expression, such as shRNA, CRISPR/Cas9 knockout, aptamers, inhibitors and the like are expected to be new approaches for cancer treatment. However, due to the complex heat shock protein family in eukaryotes, and the certain functional compensation among members of the heat shock protein family, other members of the HSPs family may compensate for the effect of HSP70 while the above methods reduce the expression of HSP70.

Part of the overexpressed HSP70 in tumor cells can be translocated to the cell membrane (mHSP70). The intracellular HSP70 promotes the occurrence and development of tumors by maintaining tumor cell homeostasis, stabilizing cyclin D1, and inhibiting apoptosis caused by oncogenes, while the HSP70 on the cell membrane surface can be recognized by immune cells. Tumor cell lines with high expression of membrane HSP70 (mHSP70) can be killed by CD56bright/CD94+NK cells in the presence of pro-inflammatory cytokines. In the field of tumor immunotherapy, T cells, antigen-presenting cells (APCs), NK cells, etc. can exert anti-tumor effects through mHSP70. In addition, mHSP70 activates dendritic cells by binding to TRL4. Compared with mHSP70 negative tumor cells, tumor cells with high expression of mHSP70 are more likely to be killed by IL-2-activated NK cells. In 2015, a phase II clinical trial was conducted to investigate the effects of TKD and IL-2 activated NK cells on non-small cell lung cancer patients. The above results indicate that mHSP70 is an important target molecule for tumor treatment.

### Lectin-like Oxidized Low-density Lipoprotein Receptor 1 (LOX1)

LOX1 is a low-density lipoprotein receptor of the C-type lectin superfamily, expressed in various cell types, including endothelial cells, platelets, macrophages, and smooth muscle cells. Human LOX1 is composed of a short N-terminal cytoplasmic domain, transmembrane domain, neck domain, and C-type lectin-like domain (CTLD). This gene is regulated through the cAMP signaling pathway, encoding proteins that bind, internalize, and degrade oxidized low-density lipoprotein (oxLDL). This protein may be involved in the regulation of Fas-induced cell apoptosis, and therefore has the function as a scavenger receptor. OxLDL is a marker of atherosclerosis, which can induce the activation and dysfunction of vascular endothelial cells, leading to pro-inflammatory reaction, pro-oxidative disease and apoptosis. The association of LOX1 and oxLDL induces the activation of NF-κB by increasing the production of intracellular reactive oxygen and various pro-atherosclerotic cellular responses, including reducing nitric oxide (NO) release, monocyte adhesion and apoptosis, and aggravate the local inflammatory response of atherosclerotic plaque by regulating a variety of gene expression of inflammatory mediators such as TNF-α and IL-6 and cell proliferation, thus promoting the development of atherosclerosis. Blocking LOX1 significantly inhibited atherosclerosis at both cellular and animal levels.

Besides oxLDL, the binding proteins of LOX1 also include oxidative modified carrier proteins, phosphatidylserine, polyanions, platelets, C-reactive proteins, lipopolysaccharides, heat shock proteins, etc. Experimental data has shown that the specific binding of LOX1 to mHSP70, mHSP60, mHSP90 affects the antigen presentation of HSP members.

Based on this, in the present invention, LOX1 fragments are integrated into CAR vectors through genetic engineering for the first time, and used to modify relevant immune cells to achieve cell specific killing of LOX1-binding protein positive cells, which can be used for the treatment of related diseases.

### Chimeric antigen receptor (CAR) of the present invention

Chimeric antigen receptor (CAR) is composed of extracellular antigen recognition region, transmembrane region, and intracellular co-stimulatory signal region.

The design of CARs has gone through the following process. The first generation CAR has only one intracellular signal component, CD3ζ or FcyRI molecule. Because there is only one activation domain in the cell, it can only cause transient T cell proliferation and less cytokine secretion, and does not provide long-term T cell proliferation signals and sustained antitumor effects *in vivo.* Therefore, it has not achieved very good clinical efficacy. In the second generation CAR, based on the original structure, a co-stimulatory molecule such as CD28, 4-1BB, OX40 and ICOS is introduced. Compared with the first generation CAR, the function has been greatly improved, and the sustainability of CAR-T cells and the ability to kill tumor cells are further enhanced. Based on the second generation CAR, some new immune stimulatory molecules such as CD27 and CD134 are linked in tandem to develop the third and fourth generation CARs.

The extracellular segment of CAR can recognize a specific antigen, then the signal is transduced through the intracellular domain to induce cell activation, proliferation, cytotoxicity, and secretion of cytokines, thereby clearing target cells. Firstly, the patient's autologous cells (or cells from heterologous donors) are isolated, activated and genetically modified to produce CAR immune cells, and then injected into the same patient's body. This method has an extremely low probability of developing graft-versus-host disease, and the antigen is recognized by immune cells in a non-MHC restricted manner.

CAR immunotherapy has achieved a very high clinical response rate in the treatment of hematological malignancies, which is unmatched by any previous treatment method and has sparked a wave of clinical research worldwide.

Specifically, the chimeric antigen receptor (CAR) of the present invention comprises an extracellular domain, a transmembrane domain, and an intracellular domain.

The extracellular domain comprises a target-specific binding element. The extracellular domain can be an ScFv based on antigen-antibody specific binding, or a natural sequence based on ligand-receptor specific binding or a derivative thereof.

In the present invention, the extracellular domain of the chimeric antigen receptor is a LOX1 protein or fragment thereof that can specifically bind to the heat shock protein target of the CAR of the present invention. More preferably, the extracellular binding domain of the chimeric antigen receptor of the present invention has an amino acid sequence of positions 58-273 of the sequence shown in SEQ ID NO: 1.

The intracellular domain includes a co-stimulatory signaling region and a ζ chain. The co-stimulatory signaling region refers to a part of the intracellular domain that includes a co-stimulatory molecule. The co-stimulatory molecule is a cell surface molecule required for efficient response of lymphocytes to antigens, rather than a antigen receptor or its ligand.

A linker (or linking peptide) can be incorporated between the extracellular domain and the transmembrane domain of the CAR, or between the cytoplasmic domain and the transmembrane domain of the CAR. As used herein, the term "linker" generally refers to any oligopeptide or polypeptide that plays a role of linking the transmembrane domain to the extracellular domain or the cytoplasmic domain in a polypeptide chain. The linker may comprise 0-300 amino acids, preferably 2-100 amino acids and most preferably 3-50 amino acids.

When the CAR of the present invention is expressed in T cell, antigen recognition can be performed based on antigen binding specificity. When the CAR binds to its associated antigen, it affects tumor cell, causing tumor cell to fail to grow, to death or to be affected otherwise, causing the patient's tumor burden to shrink or eliminate. The antigen binding domain is preferably fused to one or more intracellular domains of the co-stimulatory molecule and the ζ chain. Preferably, the antigen binding domain is fused with an intracellular domain of a combination of an ICOS and/or 4-1BB signaling domain and a CD3ζ signaling domain.

In the present invention, the extracellular domain of the CAR of the present invention also includes conservative variants thereof based on the sequence, which means that compared with the amino acid sequence of positions 58-273 of SEQ ID NO: 1, there are at most 10, preferably at most 8, more preferably at most 5, most preferably at most 3 amino acids replaced by amino acids with the same or similar properties to form a polypeptide.

In the present invention, the number of added, deleted, modified, and/or substituted amino acids is preferably not more than 40% of the total number of amino acids in the initial amino acid sequence, more preferably not more than 35%, more preferably 1-33%, more preferably 5-30%, more preferably 10-25% and more preferably 15-20%.

In he present invention, the number of added, deleted, modified and/or substituted amino acids is typically 1, 2, 3, 4, or 5, preferably 1-3, preferably 1-2, and preferably 1.

As for the hinge region and the transmembrane region (transmembrane domain), the CAR can be designed to comprise a transmembrane domain fused to the extracellular domain of the CAR. In one embodiment, a transmembrane domain that is naturally associated with one of the domains in the CAR is used. In some embodiments, transmembrane domains may be selected or modified by amino acid substitutions to avoid binding such domains to the transmembrane domain of the same or different surface membrane proteins, thereby minimizing the interaction with other members of the receptor complexes.

The intracellular domain in the CAR of the present invention comprises the 4-1BB co-stimulatory domain and the signaling domain of CD3ζ.

In one embodiment of the present invention, the CAR can specifically target mHSP70.

### Chimeric antigen receptor immune cell (CAR-immune cell)

In the present invention, it provides a chimeric antigen receptor immune cell comprising a chimeric antigen receptor of the present invention that specifically targets a LOX1 receptor (preferably mHSP70).

The chimeric antigen receptor immune cell of the present invention can be a CAR-T cell, a CAR-NK cell, or a CAR-macrophage. Preferably, the chimeric antigen receptor immune cell of the present invention is a CAR-T cell.

As used herein, the terms "CAR-T cell", "CAR-T", and "CAR-T cell of the present invention" all refer to the CAR-T cell described in the fifth aspect of the present invention.

CAR-T cells have the following advantages over other T cell-based therapies: (1) the action process of CAR-T cells is not limited by MHC; (2) given that many tumor cells express the same tumor markers, a CAR gene targeting a particular tumor marker, once being completely constructed, can be widely utilized; (3) CARs can utilize both tumor protein markers and non-protein markers of carbohydrates and lipids, expanding the target range of tumor markers; (4) the use of patient autologous cells reduces the risk of rejection reactions; (5) CAR-T cells have immune memory function and can survive in the body for a long time.

As used herein, the terms "CAR-NK cell", "CAR-NK", and "CAR-NK cell of the present invention" all refer to the CAR-NK cell described in the fifth aspect of the present invention. The CAR-NK cells of the present invention can be used in tumors with high expression of LOX1 receptors (preferably mHSP70).

Natural killer (NK) cells are a major type of immune effector cells that protect the body from viral infections and tumor cell invasion through non antigen-specific pathways. Engineered (genetic modified) NK cells may acquire new functions, including the ability to specifically recognize tumor antigens and enhanced anti-tumor cytotoxicity.

Compared with CAR-T cells, CAR-NK cells also have advantages such as: (1) directly killing tumor cells by releasing perforin and granzyme, without killing normal cells in the body; (2) releasing a small amount of cytokines, thereby reducing the risk of cytokine storms; (3) extremely easiness to be expanded and developed into "ready-made" products *in vitro.* In addition, it is similar to CAR-T cell therapy.

### Vector

The nucleic acid sequences coding for the desired molecules can be obtained using recombinant methods known in the art, such as, for example by screening libraries from cells expressing the gene, by deriving the gene from a vector known to include the same, or by isolating directly from cells and tissues containing the same, using standard techniques. Alternatively, the gene of interest can be produced synthetically.

The present invention also provides vectors comprising the nucleic acid molecule of the present invention. Vectors derived from retroviruses such as the lentivirus are suitable tools to achieve long-term gene transfer since they allow long-term, stable integration of a transgene and its propagation in daughter cells. Lentiviral vectors have the advantage over vectors derived from onco-retroviruses such as murine leukemia viruses in that they can transduce non-proliferating cells, such as hepatocytes. They also have the advantage of low immunogenicity.

In brief, the expression cassette or nucleic acid sequence of the present invention is typically and operably linked to a promoter, and incorporated into an expression vector. The vectors can be suitable for replication and integration in eukaryotes. Typical cloning vectors contain transcription and translation terminators, initiation sequences, and promoters useful for regulation of the expression of the desired nucleic acid sequence.

The expression constructs of the present invention may also be used for nucleic acid immune and gene therapy, using standard gene delivery protocols. Methods for gene delivery are known in the art. See, e.g., U.S, Pat. Nos. 5,399,346, 5,580,859, and 5,589,466, which are incorporated by reference herein in entirety. In another embodiment, the present invention provides a gene therapy vector.

The nucleic acid can be cloned into various vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al, (2001, Molecular Cloning: A Laboratory Manual, Cold Spring Harbor Laboratory, New York), and in other virology and molecular biology manuals. Viruses which are useful as vectors include but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S, Pat. No. 6,326, 193).

A number of viral based systems have been developed for transferring a gene into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either *in vivo* or *ex vivo.* A number of retroviral systems are known in the art. In some embodiments, adenovirus vectors are used. A number of adenovirus vectors are known in the art. In one embodiment, lentiviral vectors are used.

Additional promoter elements, e.g., enhancers, regulate the frequency of transcriptional initiation. Typically, these are located in the region 30-110 bp upstream of the start site, although a number of promoters have recently been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription.

One example of a suitable promoter is the immediate early cytomegalovirus (CMV) promoter sequence. This promoter sequence is a strong constitutive promoter sequence capable of driving high levels of expression of any polynucleotide sequence operatively linked thereto. Another example of a suitable promoter is Elongation Growth Factor-1α (EF-1α). However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the hemoglobin promoter, and the creatine kinase promoter. Further, the present invention should not be limited to the use of constitutive promoters. Inducible promoters are also contemplated as part of the present invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionein promoter, a glucocorticoid promoter, a progesterone promoter, and a tetracycline promoter.

In order to assess the expression of a CAR polypeptide or portions thereof, the expression vector to be introduced into a ceil can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co-transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). In one embodiment of the present invention, the reporter gene is a gene encoding the mKate2 red fluorescent protein. Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

Methods of introducing and expressing genes into a cell are known in the art. In the context of an expression vector, the vector can be readily introduced into a host cell, e.g., mammalian, bacterial, yeast, or insect cell by any method in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al. (2001, Molecular Cloning: A Laboratory Manual, Cold Spring Harbor Laboratory, New York). A preferred method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat, Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle *in vitro* and *in vivo* is a liposome (e.g., an artificial membrane vesicle).

In the case where a non-viral delivery system is utilized, an exemplary delivery vehicle is a liposome. The use of lipid formulations is contemplated for the introduction of the nucleic acids into a host cell (*in vitro, ex vivo* or *in vivo*). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

In a preferred embodiment of the present invention, the vector is a lentiviral vector.

### Formulation

The present invention provides a formulation (or preparation) comprising the chimeric antigen receptor (CAR) according to the first aspect of the present invention, the nucleic acid molecule according to the second aspect of the present invention, the vector according to the third aspect of the present invention, or the host cell according to the fourth aspect of the present invention or the engineered immune cell according to the fifth aspect of the present invention, and a pharmaceutically acceptable carrier, diluent or excipient. In one embodiment, the formulation is a liquid preparation. Preferably, the formulation is an injection. Preferably, the concentration of the CAR-T cells in the formulation is 1×10³-1×10⁸ cells/ml, more preferably 1×10⁴-1×10⁷ cells/ml.

In one embodiment, the formulation may comprises buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. The formulation of the present invention is preferably formulated for intravenous administration.

### Therapeutic application

The present invention comprises therapeutic applications by using cells (e.g., T cells) transduced with a lentiviral vector (LV) encoding the expression cassette of the present invention. The transduced T cells can target the tumor cell marker, heat shock protein (preferably mHSP70), synergistically activate immune cells, and cause T cell immune responses, thereby significantly increasing the killing efficiency against tumor cells.

Therefore, the present invention also provides a method for stimulating a T cell-mediated immune response to a target cell population or tissue in a mammal comprising a step of administering to the mammal a CAR cell of the present invention.

In one embodiment, the present invention comprises a cell therapy, wherein T cells from autologous patient (or heterologous donor) are isolated, activated and genetically modified to generate CAR-T cells, and then injected into the same patient. The probability of graft-versus-host-disease in the way is extremely low, and antigens are recognized by T cells in a non-MHC-restricted manner. In addition, one CAR-T can treat all cancers that express the antigen. Unlike antibody therapy, CAR-T cells are able to replicate *in vivo* and result in long-term persistence that can lead to sustained tumor control.

In one embodiment, the CAR-T cells of the present invention can undergo robust *in vivo* T cell expansion and can persist for an extended period. In addition, the CAR mediated immune response may be part of an adoptive immunotherapy approach in which CAR-modified T cells induce an immune response specific to the antigen binding moiety in the CAR. For example, an anti-heat shock protein (preferably mHSP70) CAR-T cell elicits an immune response specific against cells expressing heat shock protein (preferably mHSP70).

Although the data disclosed herein specifically disclose lentiviral vector comprising LOX1 protein of a fragment thereof, hinge and transmembrane domains, and 4-1BB and CD3ζ signaling domains, it is contemplated that the present invention include any number of variations for each of the components of the construct as described elsewhere herein.

Cancers that may be treated include tumors that are unvascularized or largely unvascularized, and tumors that are vascularized. Cancers may include non-solid tumors (such as hematological tumors, for example, leukemias and lymphomas) or solid tumors. Types of cancers to be treated with the CARs of the present invention include, but are not limited to, carcinoma, blastoma, and sarcoma, and certain leukemia or lymphoid malignancies, benign and malignant tumors, and malignancies e.g., sarcomas, carcinomas, and melanomas. Adult tumors/cancers and pediatric tumors/cancers are also included.

Hematologic cancers are cancers of the blood or bone marrow. Examples of hematological (or hematogenous) cancers include leukemias, including acute leukemias (such as acute lymphocytic leukemia, acute myelocytic leukemia, acute myelogenous leukemia and myeloblasts, promyeiocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

Solid tumors are abnormal masses of tissue that usually do not contain cysts or liquid areas. Solid tumors can be benign or malignant. Different types of solid tumors are named for the type of cells that form them (such as sarcomas, carcinomas, and lymphomas). Examples of solid tumors (such as sarcomas and carcinomas) include fibrosarcoma, myxosarcoma, liposarcoma, mesothelioma, malignant lymphoma, pancreatic cancer, ovarian cancer, breast cancer, gastric cancer, hepatobiliary cancer, colorectal cancer, bladder cancer, non-small cell lung cancer, ovarian and esophageal cancer, glioblastoma, and lung cancer.

The CAR-modified T cells of the present invention may also serve as a type of vaccine for *ex vivo* immunization and/or *in vivo* therapy in a mammal. Preferably, the mammal is a human.

With respect to *ex vivo* immunization, at least one of the following occurs *in vitro* prior to administering the cells into a mammal: i) expanding the cells, ii) introducing a nucleic acid encoding a CAR to the cells, and/or iii) cryopreservation of the cells.

*Ex vivo* procedures are well known in the art and are discussed more fully below. Briefly, cells are isolated from a mammal (preferably a human) and genetically modified (i.e., transduced or transfected *in vitro*) with a vector expressing a CAR disclosed herein. The CAR-modified cell can be administered to a mammalian recipient to provide a therapeutic benefit. The mammalian recipient may be a human and the CAR-modified cell can be autologous with respect to the recipient. Alternatively, the cells can be allogeneic, syngeneic or xenogeneic with respect to the recipient.

In addition to using a cell-based vaccine in terms of *ex vivo* immunization, the present invention also provides compositions and methods for *in vivo* immunization to elicit an immune response directed against an antigen in a patient.

The present invention provides a method for treating tumors comprising administering to a subject in need thereof, a therapeutically effective amount of the CAR-modified T cells of the present invention.

The CAR-modified T cells of the present invention may be administered either alone, or as a pharmaceutical composition in combination with diluents and/or with other components such as IL-2, IL-17 or other cytokines or cell populations. Briefly, pharmaceutical compositions of the present invention may comprise a target cell population as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for intravenous administration.

Pharmaceutical compositions of the present invention may be administered in a manner appropriate to the disease to be treated (or prevented). The quantity and frequency of administration will be determined by such factors as the condition of the patient, and the type and severity of the patient's disease, although appropriate dosages may be determined by clinical trials.

When "an effective amount", "an immunologically effective amount", "an anti-tumor effective amount", "an tumor-inhibiting effective amount", or "a therapeutic amount" is indicated, the precise amount of the compositions of the present invention to be administered can be determined by a physician with consideration of individual differences in age, weight, tumor size, extent of infection or metastasis, and condition of the patient (subject). It can generally be stated that a pharmaceutical composition comprising the T cells described herein may be administered at a dosage of 10⁴ to 10⁹ cells/kg body weight, preferably 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al,, New Eng. J. of Med. 319: 1676, 1988). The optimal dosage and treatment regime for a particular patient can readily be determined by one skilled in the art of medicine by monitoring the patient for signs of disease and adjusting the treatment accordingly.

The administration of the subject compositions may be carried out in any convenient manner, including by aerosol inhalation, injection, ingestion, transfusion, implantation or transplantation. The compositions described herein may be administered to a patient subcutaneously, intradermaliy, intratumorally, intranodally, intramedullary, intramuscularly, by intravenous (i.v.) injection, or intraperitoneally. In one embodiment, the T cell compositions of the present invention are administered to a patient by intradermal or subcutaneous injection. In another embodiment, the T cell compositions of the present invention are preferably administered by i.v. injection. The compositions of T cells may be injected directly into a tumor, lymph node, or site of infection.

In certain embodiments of the present invention, cells activated and expanded using the methods described herein, or other methods known in the art where T cells are expanded to therapeutic levels, are administered to a patient in conjunction with (e.g., before, simultaneously or following) any number of relevant treatments, including but not limited to treatment with agents such as antiviral therapy, cidofovir and interleukin-2, Cytarabine (also known as ARA-C) or natalizumab treatment for MS patients or efalizumab treatment for psoriasis patients or other treatments for specific tumor patients. In further embodiments, the T cells of the present invention may be used in combination with chemotherapy, radiation, immunosuppressive agents, such as cyclosporin, azathioprine, methotrexate, mycophenolate, and FK506, antibodies, or other immunotherapeutic agents. In a further embodiment, the cell compositions of the present invention are administered to a patient in conjunction with (e.g., before, simultaneously or following) bone marrow transplantation, or the use of chemotherapy agents such as, fludarabine, external-beam radiation therapy (XRT), cyclophosphamide. For example, in one embodiment, subjects may undergo standard treatment with high dose chemotherapy followed by peripheral blood stem cell transplantation. In certain embodiments, following the transplant, subjects receive an infusion of the expanded immune cells of the present invention. In an additional embodiment, expanded cells are administered before or following surgery.

The dosage of the above treatments to be administered to a patient will vary with the precise nature of the condition being treated and the recipient of the treatment. The scaling of dosages for human administration can be performed according to practices in the field. In general, 1×10⁶ to 1×10¹⁰ of CAR-T cells of the present invention can be applied to patients by means of, for example, intravenous infusion in each treatment or each course of treatment.

### Main advantages of the present invention include:

1) Target specificity: taking mHSP70 as an example, it is not expressed on the cell membrane of normal cells, but can translocate to the cell membrane under pressure stress (such as in tumors). Therefore, the present CAR only targets malignant cells with high expression of HSP70 on the cell membrane and has no killing effect on normal cells.
2) The present invention utilizes a ligand-receptor binding mechanism instead of the traditional scFv. Safety tests in animals, especially primates, better reflect their safety in the human body.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

**Table A. Summary of amino acid sequences involved in the present invention**

| Name of sequence | Sequence | SEQ ID NO: |
|---|---|---|
| Full-length LOX1 prot ein | MTFDDLKIQTVKDQPDEKSNGKKAKGLQFLYSPWWC LAAATLGVLCLGLVVTIMVLGMQLSQVSDLLTQEQA NLTHQKKKLEGQISARQQAEEASQESENELKEMIETL ARKLNEKSKEQMELHHQNLNLQETLKRVANCSAPCP QDWIWHGENCYLFSSGSFNWEKSQEKCLSLDAKLLKI NSTADLDFIQQAISYSSFPFWMGLSRRNPSYPWLWED GSPLMPHLFRVRGAVSQTYPSGTCAYIQRGAVYAENC ILAAFSICQKKANLRAQ | 1 |
| mKate2 red fluorescent protein | MSELIKENMHMKLYMEGTVNNHHFKCTSEGEGKPYE GTQTMRIKAVEGGPLPFAFDILATSFMYGSKTFINHTQ GIPDFFKQSFPEGFTWERVTTYEDGGVLTATQDTSLQ DGCLIYNVKIRGVNFPSNGPVMQKKTLGWEASTETLY PADGGLEGRADMALKLVGGGHLICNLKTTYRSKKPA KNLKMPGVYYVDRRLERIKEADKETYVEQHEVAVAR YCDLPSKLGHKLN | 2 |
| Signal peptide | MAAATGPSFWLGNETLKVPLAL | 3 |
| CD8 hinge region | TTTPAPRPPTPAPTIASQPLSLRPEACRPAAGGAVHTR GLDFACD | 4 |
| CD28 transmembr ane region | IYIWAPLAGTCGVLLLSLVITLYC | 5 |
| Co-stimulat ory signaling molecule derived from 4-1BB | KRGRKKLLYIFKQPFMRPVQTTQEEDGCSCRFPEEEE GGCELR | 6 |
| Cytoplasmi c signal transduction sequence derived from CD3ζ | VKFSRSADAPAYKQGQNQLYNELNLGRREEYDVLDK RRGRDPEMGGKPRRKNPQEGLYNELQKDKMAEAYS EIGMKGERRRGKGHDGLYQGLSTATKDTYDALHMQ ALPPR | 7 |
| Preferred full-length | MQLSQVSDLLTQEQANLTHQKKKLEGQISARQQAEE ASQESENELKEMIETLARKLNEKSKEQMELHHQNLNL | 8 |
| CAR of the present invention | QETLKRVANCSAPCPQDWIWHGENCYLFSSGSFNWE KSQEKCLSLDAKLLKINSTADLDFIQQAISYSSFPFWM GLSRRNPSYPWLWEDGSPLMPHLFRVRGAVSQTYPS GTCAYIQRGAVYAENCILAAFSICQKKANLRAQTTTP APRPPTPAPTIASQPLSLRPEACRPAAGGAVHTRGLDF ACDIYIWAPLAGTCGVLLLSLVITLYCKRGRKKLLYIF KQPFMRPVQTTQEEDGCSCRFPEEEEGGCELRVKFSR SADAPAYKQGQNQLYNELNLGRREEYDVLDKRRGR DPEMGGKPRRKNPQEGLYNELQKDKMAEAYSEIGMK GERRRGKGHDGLYQGLSTATKDTYDALHMQALPPR | |
| Nucleotide sequence encoding the preferred full-length CAR of the present invention | atgcaattatcccaggtgtctgacctcctaacacaagagcaagcaaacctaactcac cagaaaaagaaactggagggacagatctcagcccggcaacaagcagaagaagct tcacaggagtcagaaaacgaactcaaggaaatgatagaaacccttgctcggaagct gaatgagaaatccaaagagcaaatggaacttcaccaccagaatctgaatctccaag aaacactgaagagagtagcaaattgttcagctccttgtccgcaagactggatctggc atggagaaaactgttacctattttcctcgggctcatttaactgggaaaagagccaaga gaagtgcttgtctttggatgccaagttgctgaaaattaatagcacagctgatctggact tcatccagcaagcaatttcctattccagttttccattctggatggggctgtctcggagg aaccccagctacccatggctctgggaggacggttctcctttgatgccccacttattta gagtccgaggcgctgtctcccagacatacccttcaggtacctgtgcatatatacaac gaggagctgtttatgcggaaaactgcattttagctgccttcagtatatgtcagaagaa ggcaaacctaagagcacagaccacgacgccagcgccgcgaccaccaacaccgg cgcccaccatcgcTAGCcagcccctgtccctgcgcccagaggcgtgccggcc agcggcggggggcgcagtgcacacgagggggctggacttcgcctgtgatatcta catctgggcgcccttggccgggacttgtggggtccttctcctgtcactggttatcacc ctttactgcaaacggggcagaaagaaactcctgtatatattcaaacaaccatttatga gaccagtacaaactactcaagaggaagatggctgtagctgccgatttccagaagaa gaagaaggaggatgtgaactgagagtgaagttcagcaggagcgcagacgccccc gcgtacaagcagggccagaaccagctctataacgagctcaatctaggacgaagag aggagtacgatgttttggacaagagacgtggccgggaccctgagatggggggaa agccgagaaggaagaaccctcaggaaggcctgtacaatgaactgcagaaagata agatggcggaggcctacagtgagattgggatgaaaggcgagcgccggaggggc aaggggcacgatggcctttaccagggtctcagtacagccaccaaggacacctacg acgcccttcacatgcaggccctgccccctcgc | 9 |

### Example 1: Detection of HSP70 Localization in Pancreatic Cancer Sample Chip

A pancreatic cancer sample microarray was purchased from Shanghai Outdo Biotech, containing 23 pairs of adjacent non-cancerous and cancerous tissue samples, as well as 14 cases of pancreatic cancer samples. Immunohistochemical staining was used to detect the expression of HSP70 (red) and the cell membrane marker β-catenin (green). Laser confocal microscopy was used to capture images and IMAGE J software was utilized to analyze the co-localization of the two markers.

The results of this example are shown in Fig. 1. The results demonstrates that, compared to adjacent non-cancerous tissues, the co-localization ratio of HSP70 and β-catenin is higher in cancerous tissues, indicating that HSP70 is localized on the cell membrane in cancerous tissues.

### Example 2: Preparation of LOX1-CAR Vector

The nucleotide sequence of LOX1 (NM_001172632.2) and gene sequence information of human CD8α hinge region, human CD8 transmembrane region, human 4-1BB intracellular region, and human CD3ζ intracellular region were obtained from the NCBI database. The CD8 signal peptide and the extracellular domain of LOX1 were synthesized by BGI, and the nucleotide sequence of the CAR molecule was digested with XbaI (Thermo) and NheI (Thermo), ligated by T4 DNA ligase (NEB) and inserted into the lentiviral vector pTomo, which had already been inserted with CD8TM, 4-1BB, and CD3ζ. The vector was transformed into competent E. coli (Stbl3).

The recombinant plasmid was sent to TsingKe Biotechnology Co., Ltd. for sequencing, and the sequencing results were aligned to confirm the correctness of the plasmid. The sequencing primer used was the universal sequencing primer CMV-F: CGCAAATGGGCGGTAGGCGTG.

All plasmids were extracted using the endotoxin-free large-scale extraction kit from QIAGEN. The purified plasmids were used to transfect 293T cells with Beyotime lipo6000 for lentivirus packaging.

The results of this example are shown in Fig. 2.

### Example 3: Packaging Virus

Packaging was performed in a 15 cm dish using 293T cells less than 20 passages old. Transfection was carried out when the confluence of 293T cells was about 80%-90%. A plasmid mixture dissolved in 2ml OPTIMEM was prepared (core plasmid 20ug, pCMVΔR8.9 10ug, PMD2.G 4ug); and 2ml OPTIMEM and 68ul of lipo 6000 were combined in another tube. After set at room temperature for 5 minutes, the plasmid complex was added to the liposome complex and set at room temperature for 20 minutes. The mixture was then added dropwise to the 293T cells and incubated at 37°C for 6 hours before removing the medium. Pre-warmed complete media were added. Viral supernatants were collected at 48 hours and 72 hours post-transfection, centrifuged at 3000 rpm for 20 minutes at 4°C, filtered through a 0.45um filter membrane, and then concentrated by centrifugation at 25000rpm for 2.5 hours at 4°C. The concentrated virus was dissolved overnight in 30 µl of virus dissolving solution, and the virus titer was detected by QPCR.

### Example 4: Preparation of CART cells

Mononuclear cells were isolated from human peripheral blood using Ficoll separation solution, and purified CD3+ T cells were obtained using RosetteSep Human T Cell Enrichment Cocktail (Stemcell technologies). T cells were activated with CD3/CD28 magnetic beads (Life technology) and stimulated and cultured with 200U/ml of IL2 (PeproTech) added for 48 hours before viral infection. Lentivirus was used to infected T cells at an MOI=20 in the presence of lentiboost to prepare CART cells. The medium was replaced after one-day infection.

### Example 5: Detection of Positive Rate of CART Cells Infection by Flow Cytometry

CART cells and NT cells (control group) were centrifuged and collected separately after 72 hours of virus infection. They were then washed once with PBS, and the supernatant was discarded. The cells were resuspended in PBS containing 2% FBS, and the positive rate was detected by flow cytometry.

The results of the transfection efficiency are shown in Fig. 3. The results indicate that the positive rate of CART cells is approximately 35%.

### Example 6: Detection of mHSP70 Expression in Various Target Cells

Target cells were seeded onto round slips of a 24-well plate and washed three times with PBST for 5 minutes each after 24 hours. They were then incubated with an antibody specific recognizing mHSP70 at room temperature for 1 hour. The cells were fixed with 4% PFA for 20 minutes and blocked with 10% goat serum at room temperature for 1 hour. The following day, the cells were washed three times with PBST for 5 minutes each. Cells were added with a secondary antibody that specific recognizes mHSP70 and is labeled with CY3, and incubated at room temperature for 1 hour. The cells were washed three times with PBS, and the nuclei were stained with DAPI. Confocal microscopy imaging was performed.

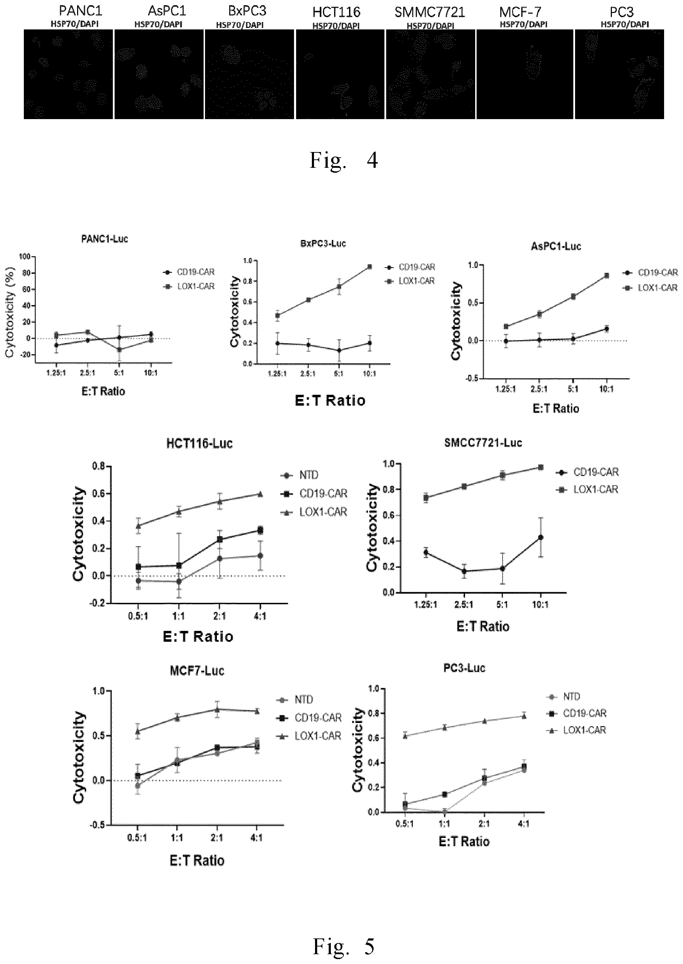

The results of this example are shown in Fig. 4. The results indicate that HSP70 is located on the cell membrane in pancreatic cancer cell lines AsPC1 and BxPC3, as well as liver cancer cell line SMMC-7721, colorectal cancer cell line HCT116, breast cancer cell line MCF-7 and prostate cancer cell line PC3, while pancreatic cancer cell line PANC1 does not express HSP70.

### Example 7: Construction of Luciferase-Expressing Target Cells

Luciferase fragment was amplified by PCR from the pGL3-luciferase plasmid, and then ligated into the pTomo vector using XbaI and BamHI to construct the pTomo-luciferase plasmid. Fragments of IRES and puromycin were amplified from the pTomo and PLkO.1 plasmids, respectively. The pTomo-luciferase-IRES-Puro plasmid was successfully constructed by ligating these three fragments.

The lentiviral packaging and titration detection were performed as described above. The virus was used to infect human embryonic kidney 293T cells, human breast cancer cell line MCF7, prostate cancer cell line PC3, colorectal cancer cell line HCT116, liver cancer cell line SMMC7721, pancreatic cancer cell lines BxPc3, PANC1, and AsPC1.

After 48 hours, the cells were selected with puromycin (1 µg/ml) for one week to obtain 293T-luciferase, MCF7-luciferase, MDA-MB231-luciferase, PC3-luciferase, SMMC7721-luciferase, HCT116-luciferase, PANC1-luciferase, Bxpc3-luciferase, and AsPC1-luciferase cells.

### Example 8: Killing Effect of CART Cells

The MCF7-luciferase cells were counted and adjusted to a density of 2×10⁴ cells/ml. 100ul of MCF7-luciferase cells were seeded into a 96-well plate, and CART/NT cells were adjusted to a density of 1×10⁵ cells. They were then seeded into a black 96-well plate at different effector to target (E:T) ratios of 0.5:1, 1:1, 2:1, and 4:1, in a volume of 100 µl per well. The above target cells and T cells were mixed and incubated in a culture incubator for 24 hours.

The supernatant was collected and stored at -80°C for the detection of IFNγ release. Cell killing was detected using the Promega fluorescence detection kit. First, the cells were lysed with 20 µl of 1*PLB lysis buffer for 20 minutes. Then, 100 µl of substrate was added to each well, and the reaction was immediately detected using a BioTek plate reader.

The cytotoxicity killing rate % = (1 - the fluorescence value of target cells with effector cells / the fluorescence value of target cells without effector cells) ×100%

The results are shown in Fig. 5. The results indicate that LOX1-CART cells have a gradually increasing killing effect on tumor cells as the E:T ratio increases.

### Example 9: Detection of IFNγ Cytokine Release

After being thawed from -80°C, the cell supernatant was used to detect IFNγ using the IFN gamma Human ELISA Kit (Life Technology).

The standard samples were dissolved using Standard Dilution Buffer, and gradientally diluted into standard samples of 1000 pg/ml, 500 pg/ml, 250 pg/ml, 125 pg/ml, 62.5 pg/ml, 31.2 pg/ml, 15.6 pg/ml, and 0 pg/ml.

To each well, 50 µl Incubation buffer, 50 µl sample to be detected and 50 µl IFNγ biotin conjugate solution were added, mixed well and incubated at room temperature for 90 minutes.

Then the following steps are performed in order:
(1) Wash the wells four times with 1* Wash Buffer, leaving each time for one minute.
(2) Add 100 µl 1* Streptavidin-HRP solution to each well and incubate at room temperature for 45 minutes.
(3) Wash the wells four times with 1* Wash Buffer, leaving each time for one minute.
(4) Add 100 µl Stabilized chromogen and incubate at room temperature for 30 minutes.
(5) Add 100 µl Stop solution to each well and mix well.
(6) Detect the absorbance at 450 nm.

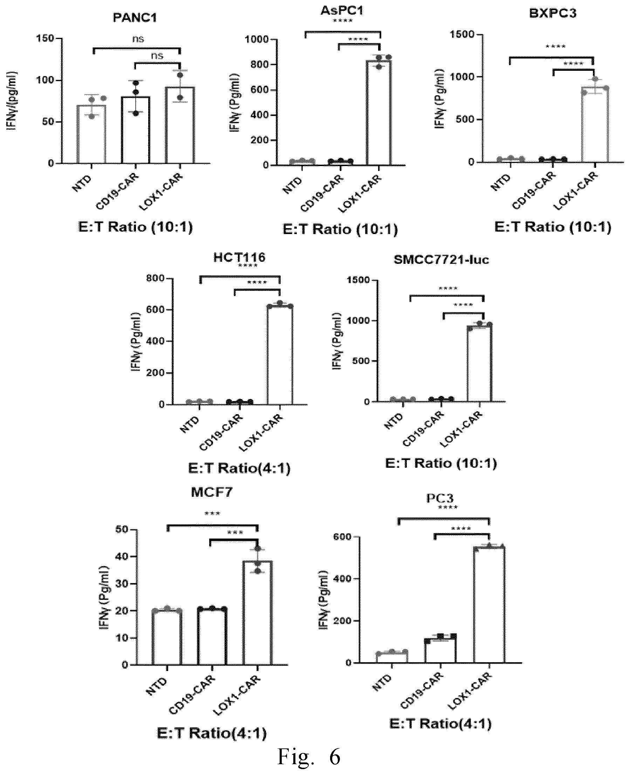

The results are shown in Fig. 6. The results indicate that the killing effect of LOX1-CART cells on tumor cells is related to the release of IFNγ.

### Example 10: Killing of LOX1-CART on Pancreatic Cancer Stem Cells

Tumor cell lines AsPC1-luc and PANC1-luc were cultured in selective medium DMEM/F12 + EGF (20 ng/ml) + bFGF (20 ng/ml) + B27 (1×) + 1% PS. After the tumor stem cells formed spheres, flow cytometry was used to detect HSP70 expression, and RNA was extracted and reverse transcribed into cDNA to detect the expression of tumor stem cell markers such as CD133, OCT4, and CXCR4. The detection of killing of tumor stem cells and cytokine release by LOX1-CART were carried out as described previously.

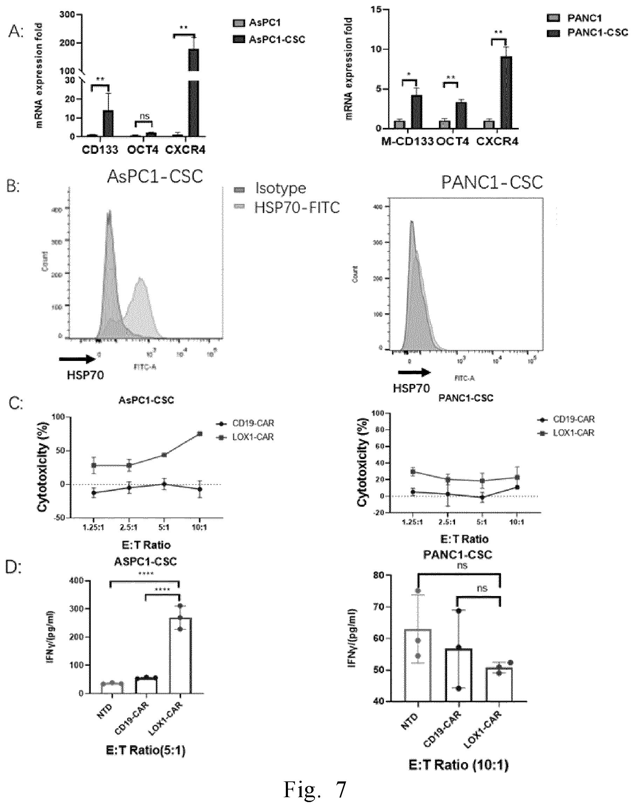

The results shown in Fig. 7 indicate that compared to AsPC1 cells, the expression of CD133 and CXCR4 in AsPC1-CSC significantly increases. Compared to PANC1 cells, the expression of CD133, OCT4, and CXCR4 in PANC1-CSC significantly increases. Moreover, AsPC1-CSC expresses hsp70, while PANC1-CSC does not express hsp70. LOX1-CART significantly kills AsPC1-CSC cells but does not kill PANC1-CSC cells, and the killing effect is positively correlated with IFNγ release.

### Example 11: Effect of Overexpression of HSP70 on LOX1-CART Tumor Killing

MDAMB231 is a mHSP70-negative triple-negative breast cancer cell line, and LOX1-CART has no killing effect on MDAMB231. Therefore, we synthesized the HSP70 coding region *in vitro* and constructed pTomo-CMV-HSP70-luciferase-IRES-puro by enzymatic cleavage and connection. The lentivirus were packaged *in vitro* and used to infect MDAMB231 cells. After puromycin (1 µg/ml) selection for 7 days, MDAMB231-HSP70 cell lines that stably expressed both HSP70 and luciferase were obtained. *In vitro* killing experiment was carried out as described previously. The killing effect of LOX1-CART on MDAMB231 and MDAMB231-HSP70 by measuring changes of fluorescence intensity.

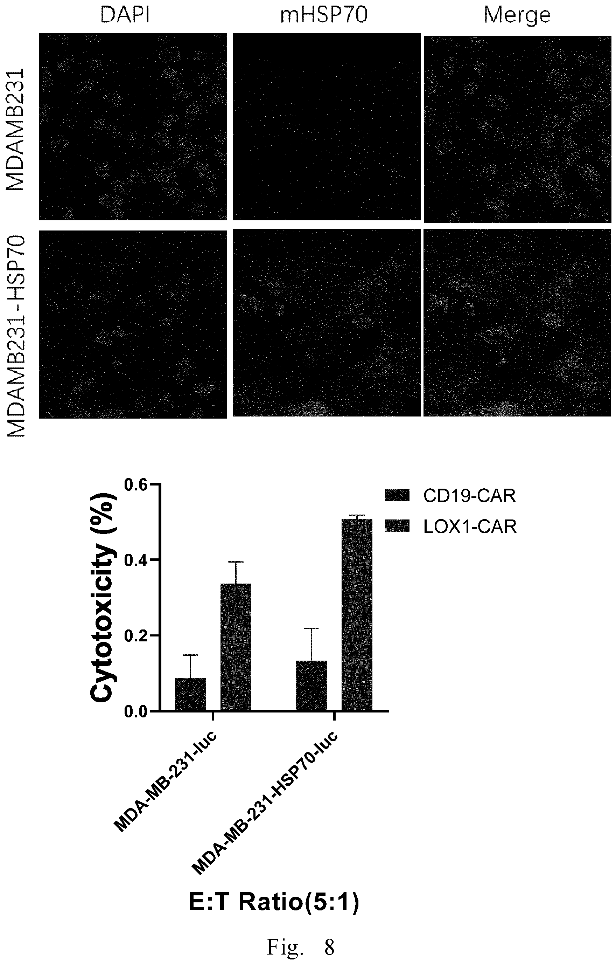

The results are shown in Fig. 8. The results indicate that the overexpression of HSP70 on the cell membrane enhances the killing effect of LOX1-CART.

### Example 12: LOX1-CART killing on normal cells

293T cells are human embryonic kidney cell lines. LOX1-CART cells were co-incubated with 293T cells at an effector to target ratio (E:T) of 5:1 and killing effect of LOX1-CART on 293T cells was detected by changes of fluorescence intensity.

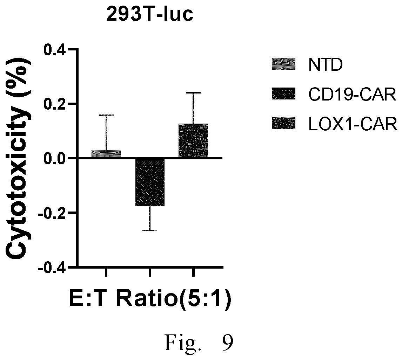

The results are shown in Fig. 9. The results indicate that LOX1-CART has no significant killing effect on 293T cells.

### Example 13: Inhibiting Effect of LOX1-CAR on ASPC1 Subcutaneous Xenograft Tumors of nude mice

AsPCl-luciferase cells were digested and counted, and the cell density was adjusted to 5×10⁶/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 µl tumor cell suspension into each mouse. Five days after tumor subcutaneous inoculation, small animal live imaging was performed and the mice were grouped as follows: NTD (uninfected T cell group, n=6), CD19-CART group (n=6), and LOX1-CART group (n=6). CD19-CART and LOX1-CART were prepared as described previously. T cells and CART cells were counted, and the density was adjusted to 1×10⁸/ml. Each mouse was injected with 100 µl T cell or CART cell suspension via the tail vein. Subsequently, small animal live imaging was performed every 7 days and changes in tumor fluorescence signals were recorded. The experiment was terminated when the tumor volume reached about 2000 mm³ and the tumor volume size was calculated.

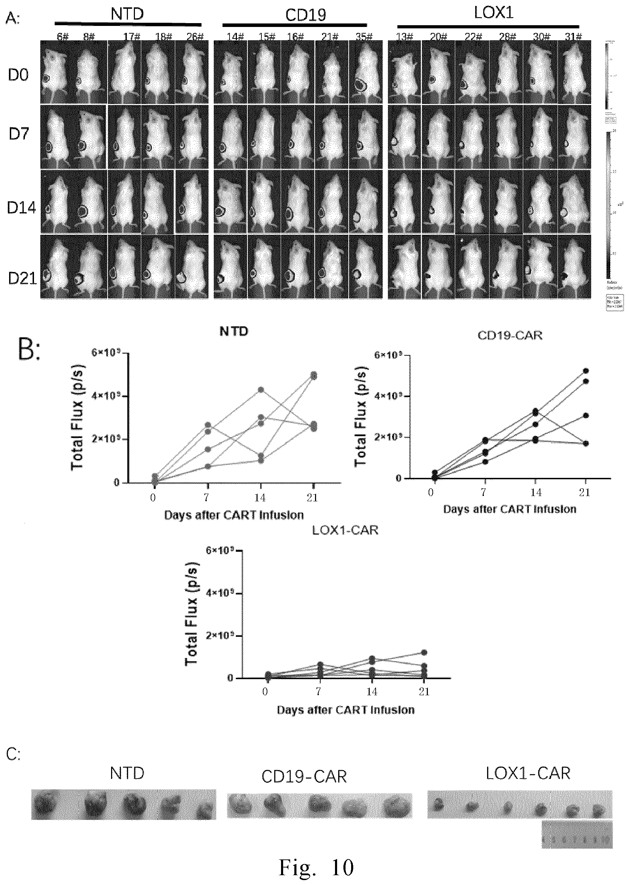

The results shown in Figure 10 indicate that compared to the control group, LOX1-CART significantly inhibited tumor growth.

### Example 14: LOX1-CART Infiltration in Mouse Organs

AsPCl-luciferase cells were digested and counted, and the cell density was adjusted to 5×10⁶/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 µl tumor cell suspension into each mouse. Five days after tumor subcutaneous inoculation, small animal live imaging was performed and the mice were grouped as follows: CD19-CART group (n=3), and LOX1-CART group (n=3). CD19-CART and LOX1-CART were prepared as described previously. CART cells were counted, and the density was adjusted to 1×10⁸/ml. Each mouse was injected with 100 µl CART cell suspension via the tail vein. Tissues (subcutaneous tumor, heart, liver, spleen, lung, kidney, pancreas, and brain) were collected on the fifth day after tumor inoculation. Part of the tissues were stained with HE, and the other part was subjected to immunohistochemistry for CD3 staining to detect CART cell infiltration.

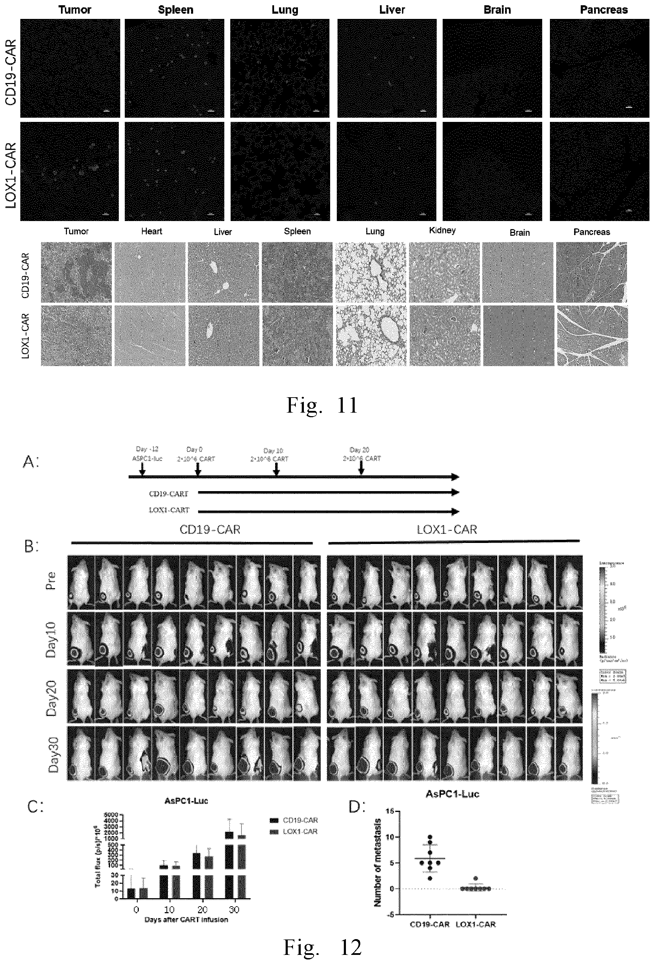

The experimental results are shown in Fig. 11. The LOX1-CAR group has a significant enrichment of T cells in the transplanted tumor while the CD19-CAR group has not, and there is no significant difference in other tissues between the 2 T cell groups. HE staining results show that both CD19-CAR and LOX1-CAR treatments do not cause significant damage to the major organs.

### Example 14: Inhibition on Pancreatic Cancer Metastasis by LOX1-CART

AsPCl-luciferase cells were digested and counted, and the cell density was adjusted to 5×10⁶/ml (containing 20% matrigel). Six-week-old female NCG mice were purchased from Nanjing Jike Pharmaceutical Biotechnology Co., Ltd. and raised in a SPF animal house for about 1 week before subcutaneous injection of 100 µl tumor cell suspension into each mouse. 12 days after tumor subcutaneous inoculation (when the tumor volume had reached a certain size), small animal live imaging was performed and the mice were grouped as follows: CD19-CART group (n=7), and LOX1-CART group (n=7). CD19-CART and LOX1-CART were prepared as described previously. T cells and CART cells were counted, and the density was adjusted to 2×10⁷/ml. Each mouse was injected with 100 µl T cell or CART cell suspension via the tail vein. Subsequently, the mice received three additional re-infusion of the same amount of CART cells every ten days. Small animal live imaging was performed simultaneously and changes in tumor fluorescence signals were recorded. Blood samples were collected from the mice at different time points before tumor cell transplantation and before and after CART cell injection, and stored at -80°C.

The experimental results are shown in Fig. 12. Multiple injections of low-dose LOX1-CART cells do not significantly inhibit subcutaneous tumor growth but suppressed the formation of lung metastatic foci.

Genome was extracted from peripheral blood using phenol-chloroform, and quantified using NanoDrop one. To detect the presence of tumor cells in peripheral blood, specific primers targeting luciferase were used for fluorescence quantification.

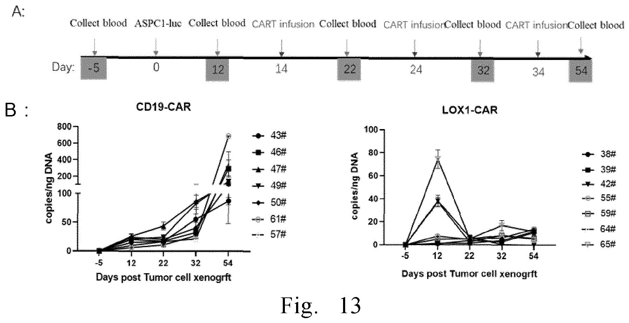

The experimental results are shown in Fig. 13. Compared to the CD19-CART group, LOX1-CART injection significantly reduced tumor cells in peripheral blood.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. A chimeric antigen receptor (CAR) modified based on C-type lectin superfamily low-density lipoprotein receptor 1 (LOX1), wherein the CAR comprises an extracellular binding domain that specifically binds to a LOX1 receptor.

2. The CAR according to claim 1, wherein the LOX1 receptor is selected from a group consisting of: a heat shock protein, oxidized low-density lipoprotein and phosphatidylserine.

3. The CAR according to claim 1, wherein the extracellular binding domain comprises a LOX1 protein or a fragment thereof which has an amino acid sequence as shown in SEQ ID NO: 1 or has an amino acid sequence as shown in positions 1-273 (preferably positions 38-273, more preferably positions 58-273, and more preferably positions 151-265) of SEQ ID NO: 1.

4. The CAR according to claim 1, wherein the amino acid sequence of the LOX1 protein or the fragment thereof is selected from a group consisting of:
(i) a sequence as shown in positions 58-273 of the sequence of SEQ ID NO: 1; and
(ii) an amino acid sequence obtained by replacing, deleting, altering or inserting one or more amino acid residues, or adding 1-30 amino acid residues, preferably 1-10 amino acid residues, more preferably 1-5 amino acid residues on the basis of the sequence shown in positions 58-273 of the sequence of SEQ ID NO: 1; wherein the obtained amino acid sequence has a sequence identity of ≥ 85% (preferably ≥ 90%, more preferably ≥ 95%, such as ≥ 96%, ≥ 97%, ≥ 98%, or ≥ 99%) with the sequence as shown in positions 58-273 of SEQ ID NO: 1; and the obtained amino acid sequence has the same or similar function as the sequence in (i).

5. The CAR according to any one of claims 1-4, wherein the structure of the chimeric antigen receptor is shown in the following Formula I:
L-EB-H-TM-C-CD3ζ-RP (I)
wherein,
each "-" is independently a linking peptide or peptide bond;
L is absent or a signal peptide sequence;
EB is an extracellular binding domain;
H is absent or a hinge region;
TM is a transmembrane domain;
C is absent or a co-stimulatory signaling molecule;
CD3ζ is a cytoplasmic signal transduction sequence derived from CD3ζ;
RP is absent or a reporter protein.

6. The CAR according to claim 5, wherein the amino acid sequence of the chimeric antigen receptor is shown in SEQ ID NO: 8.

7. A nucleic acid molecule encoding the chimeric antigen receptor according to claim 1.

8. A vector comprising the nucleic acid molecule according to claim 7.

9. A host cell comprising the vector according to claim 8, or having the exogenous nucleic acid molecule according to claim 7 integrated into the chromosome thereof, or expressing the CAR according to claim 1.

10. An engineered immune cell comprising the vector according to claim 8, or having the exogenous nucleic acid molecule according to claim 7 integrated into the chromosome thereof, or expressing the CAR according to claim 1.

11. A method for the preparation of the engineered immune cell according to claim 10, which comprises a step of transducing the nucleic acid molecule according to claim 7 or the vector according to claim 8 into the immune cell, thereby obtaining the engineered immune cell.

12. A pharmaceutical composition comprising the CAR according to claim 1, the nucleic acid molecule according to claim 7, the vector according to claim 8, the host cell according to claim 9, and/or the engineered immune cell according to claim 10, and a pharmaceutically acceptable carrier, diluent or excipient.

13. Use of the CAR according to claim 1, the nucleic acid molecule according to claim 7, the vector according to claim 8, or the host cell according to claim 9, and/or the engineered immune cell according to claim 10, in the preparation of a medicament or a formulation for preventing and/or treating a disease related to abnormal expression of LOX1 receptor.

14. The use according to claim 13, wherein the disease related to abnormal expression of LOX1 receptor comprises a disease related to abnormal expression of membrane-bound HSP70 (mHSP70).

15. A method of treating a disease which comprises: administering an effective amount of the engineered immune cell according to claim 10, or the pharmaceutical composition according to claim 12, to a subject in need thereof.
